# EUROPEAN PATENT APPLICATION

(11) **EP 3 189 799 A1**
(43) Date of publication of application: **12.07.2017**
(21) Application number: 17150347.7
(22) Date of filing: 05.01.2017
(51) Int. Cl.: A61B 17/32, A61B 1/06, A61B 17/24, A61B 90/13, A61B 17/00, A61B 90/00

(54) **OPTICAL REGISTRATION OF ROTARY SINUPLASTY CUTTER**

(30) Priority: 06.01.2016 US 201614989354
(71) Applicant: Biosense Webster (Israel), Ltd., Yokneam, 2066717 (IL)
(72) Inventor: GOVARI, Assaf, 2066717 Yokneam (IL); GLINER, Vadim, 2066717 Yokneam (IL)
(74) Representative: Carpmaels & Ransford LLP

(57) **Abstract**

A surgical apparatus includes a rotatable shaft and a hollow insertion tube. The shaft includes at least one optical marker located thereon. The insertion tube is configured for insertion into a patient body, and is coaxially disposed around the shaft. One or more optical devices are coupled around an inner perimeter of the insertion tube and are configured to detect the marker and output one or more signals indicative of an angular position of the detected marker.

## Description

### FIELD OF THE INVENTION

The present invention relates generally to minimally-invasive surgery, and specifically to registration of a rotary cutter.

### BACKGROUND OF THE INVENTION

Rotary cutters are used in a variety of minimally-invasive medical applications. Examples of prior art techniques are provided below.

U.S. Patent 7,918,784, whose disclosure is incorporated herein by reference, describes an endoscopic tool that utilizes a fiber optic system for illuminating and imaging ligaments or other tissue, which are to be cut. Illumination and imaging is performed above a lateral opening at the distal end of a probe that is inserted into an incision point. A two edged blade which can be moved both in the distal to proximal direction and in the proximal to distal direction is selectively deployable from of the lateral opening at the distal end of the probe.

U.S. Patent Application Publication 2014/0288560, whose disclosure is incorporated herein by reference, describes a surgical cutting instrument including two coaxially arranged tubular members. The first tubular member has a cutting tip and is co-axially disposed within the second tubular member, which in turn has a cutting window to expose the cutting tip to tissue and bone through the cutting window. Movement of the tubular members with respect to each other debrides soft tissue and thin bone presented to the cutting window. The second (outer) tubular member has one or more features, which contribute to reduce clogging of the cutting tip as a whole.

Documents incorporated by reference in the present patent application are to be considered an integral part of the application except that, to the extent that any terms are defined in these incorporated documents in a manner that conflicts with definitions made explicitly or implicitly in the present specification, only the definitions in the present specification should be considered.

### SUMMARY OF THE INVENTION

An embodiment of the present invention that is described herein provides surgical apparatus including a rotatable shaft and a hollow insertion tube. The shaft includes at least one optical marker located thereon. The insertion tube is configured for insertion into a patient body, and is coaxially disposed around the shaft. One or more optical devices are coupled around an inner perimeter of the insertion tube and are configured to detect the marker and output one or more signals indicative of an angular position of the detected marker.

In some embodiments, the surgical apparatus further includes a processor that is configured to receive the signals from the optical devices and to calculate based on the signals, a rotational angle of the shaft with respect to the insertion tube. In an embodiment, the processor is further configured to automatically rotate the shaft, in response to the signals received from the optical devices, so as to set a desired rotational angle of the shaft with respect to the insertion tube.

In other embodiments, the hollow insertion tube has an opening, and the rotatable shaft includes a cutter, which is configured, when the shaft is rotating, to cut an object that enters the insertion tube via the opening. In yet other embodiments, the marker is located at a predefined rotational angle with respect to the cutter.

In an embodiment, the marker faces the inner perimeter of the insertion tube. In another embodiment, each of the optical devices includes a light source and a detector, the light source is configured to illuminate the inner perimeter, and the detector is configured to detect the marker by detecting a light reflection from the inner surface. In yet another embodiment, the light source includes a light emitting diode (LED).

There is additionally provided, in accordance with an embodiment of the present invention, a method including providing a surgical tool including a rotatable shaft, which includes at least one optical marker located thereon, and a hollow insertion tube coaxially disposed around the shaft. The surgical tool is inserted into a patient body. While the surgical tool is inside the patient body, the marker is detected using one or more optical devices that are coupled around an inner perimeter of the insertion tube. The optical devices are used to output one or more signals indicative of an angular position of the detected marker.

There is further provided, in accordance with an embodiment of the present invention, a method for producing a surgical tool including providing a rotatable shaft, which includes at least one optical marker located thereon. A hollow insertion tube is coaxially disposed around the shaft, and one or more optical devices are coupled around an inner perimeter of the insertion tube.

The present disclosure will be more fully understood from the following detailed description of the embodiments thereof, taken together with the drawings, in which:

### BRIEF DESCRIPTION OF THE DRAWINGS

FIG. 1 is a schematic pictorial illustration of a surgical system, in accordance with an embodiment of the present invention;
FIG. 2A is a schematic side view of a distal end of a surgical catheter, in accordance with an embodiment of the present invention; and
FIG. 2B is a schematic sectional view of a registration module of the surgical catheter of Fig. 2A, in accordance with an embodiment of the present invention.

### DETAILED DESCRIPTION OF EMBODIMENTS

### OVERVIEW

Minimally-invasive surgery techniques are used in a variety of surgical procedures, such as sinuplasty, in which a physician removes objects or tissue such as nasal polyps. During the procedure, the physician navigates the distal end of a surgical catheter to the location of the polyp. The distal end of the catheter may comprise various kinds of surgical tools, such as a rotatable cutter disposed in an insertion tube having an opening. The physician navigates the distal end in attempt to insert the polyp into the opening, and then cuts the polyp by rotating the cutter. In some cases, e.g., after removing the polyp, it is important for the physician to know the angular position of the cutter relative to the opening, and also to be able to adjust this position.

Embodiments of the present invention that are described hereinbelow provide improved techniques for identifying the angular position of a rotatable catheter shaft relative to an insertion tube in which the shaft is rotated. These techniques can be used, for example, in a surgical sinuplasty catheter, for identifying and controlling the position of a cutter attached to the shaft, relative to an opening in the insertion tube.

In some embodiments, the rotatable shaft has one or more optical markers located thereon. In an embodiment, a marker may be located in the vicinity of the distal end of the shaft, at a predefined angular distance from the cutter. In addition, one or more optical devices, each comprising an optical emitter and an optical detector, are coupled to the inner perimeter of the insertion tube, opposite the marker. Each optical device is configured to detect the marker when facing it, and to output a corresponding signal. A processor analyzes the signals produced by the optical devices, and calculates the angular position of the marker relative to the optical devices (and thus the angular position of the cutter relative to the opening). The processor may present this angular position to the physician using a suitable interface.

### SYSTEM DESCRIPTION

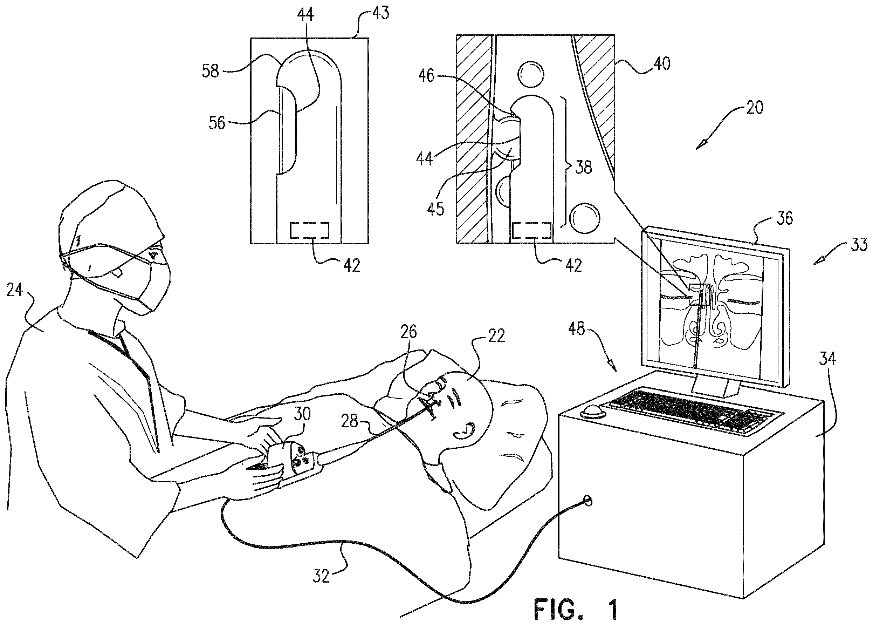

FIG. 1 is a schematic pictorial illustration of a sinuplasty procedure using a surgical system 20, in accordance with an embodiment of the present invention. System 20 comprises a catheter 28, which a physician 24 inserts into a nose 26 of a patient 22 so as to remove a foreign object or a tumor, such as a nasal polyp 45 (shown in an inset 40). Catheter 28 comprises a proximal end 30, configured to control a distal end 38 of the catheter.

System 20 further comprises a console 33, which comprises a processor 34, typically a general-purpose computer, with suitable front end and interface circuits for receiving signals from catheter 28, via a cable 32, and for controlling other components of system 20 described herein. Console 33 further comprises input devices 48 and a display 36, which is configured to display the data (e.g., images) received from processor 34 or inputs inserted by a user (e.g., physician 24).

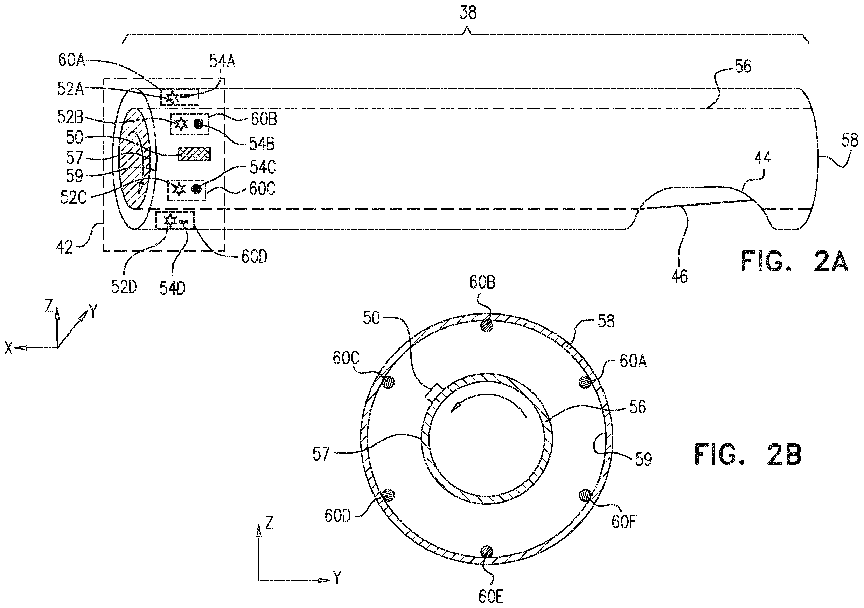

Referring to insets 40 and 43, distal end 38 typically comprises a rigid hollow insertion tube 58 for insertion into the nose of patient 22. Tube 58 is coaxially disposed around a rotating shaft 56 (shown in FIGs. 2A and 2B). Shaft 56 may be driven using any suitable mechanism, such as a direct current (DC) motor that can rotate clockwise and counterclockwise depending on the polarity of the electrical current applied to the motor.

In some embodiments, tube 58 has an opening 44. Shaft 56 comprises a sinuplasty cutter 46 that is aligned with opening 44 in the insertion tube. Cutter 46 rotates with the shaft and is configured to cut polyp 45.

Referring to inset 40, during the sinuplasty procedure, physician 24 navigates catheter 28 so that opening 44 is facing polyp 45. In an embodiment, cutter 46 does not block opening 46 so that polyp 45 may be inserted through opening 44 into tube 58. In other embodiments, physician 24 may confirm the position of opening 46 with respect to polyp 45 using mapping techniques such as depicted in U.S. patent application 14/942,455, to Govari et al., filed November 16, 2015, which is incorporated herein by reference.

Once polyp 45 passes through opening 44, physician 24 may use console 33 or proximal end 30 to rotate shaft 56 including cutter 46 so as to remove at least part of polyp 45. In some embodiments, after removing the polyp, physician 24 may rotate shaft 56 to any desired angular position relative to opening 44. For example, as shown in inset 43, physician 24 may rotate shaft 56 so that cutter 46 is facing the right side of tube 58 and the body of shaft 56 blocks opening 44. Catheter 28 draws the removed polyp into a drain (not shown) located, for example, in proximal end 30.

In some embodiments, distal end 38 further comprises a registration module 42, which is configured to assist physician 24 with controlling the rotational angle of cutter 46 relative to opening 44. Module 42 is further described in FIGs. 2A and 2B below.

Fig. 1 shows only elements related to the disclosed techniques, for the sake of simplicity and clarity. System 20 typically comprises additional modules and elements that are not directly related to the disclosed techniques, and thus, intentionally omitted from Fig. 1 and form the corresponding description.

Processor 34 may be programmed in software to carry out the functions that are used by the system, and to store data in a memory (not shown) to be processed or otherwise used by the software. The software may be downloaded to the processor in electronic form, over a network, for example, or it may be provided on non-transitory tangible media, such as optical, magnetic or electronic memory media. Alternatively, some or all of the functions of processor 34 may be carried out by dedicated or programmable digital hardware components.

### OPTICAL REGISTRATION OF ROTARY SINUPLASTY CUTTER

A sinuplasty procedure typically involves inserting the catheter and navigating it to the location of polyp 45, removing the polyp and retracting the catheter out of the patient's nose. Opening 44 is typically blocked by shaft 56 during the insertion phase so as to allow smooth navigation of distal end 38 toward polyp 45 without damaging tissue that may unintentionally enter opening 44. After removing the polyp, it is important for physician 24 to know the angular position of cutter 46 relative to opening 44, and to adjust this angular position as necessary.

For example, when physician 24 is required to remove another polyp (not shown) in the vicinity of polyp 45, the physician may rotate cutter 46 so as to insert the second polyp into opening 44 and remove it. In other cases, during retraction, physician 24 may prefer to rotate the shaft to block opening 44 so as to enable smooth retraction of catheter 28 from nose 26. During the procedure, distal end 38 is located deep in the nose of patient 22, and therefore physician 24 is unable to see the location of cutter 46 relative to opening 44. The disclosed technique overcomes this limitation by incorporating registration module 42 in catheter 28 so as to allow physician 24 to identify the location of cutter 46.

FIG. 2A is a schematic side view of distal end 38 in a direction of a y-axis, in accordance with an embodiment of the present invention. In the example of Fig. 2A, shaft 56 rotates so as to position cutter 46 at an angular position similar to the angular position of opening 44 so as to cut an object that enters the opening (e.g., polyp 45 shown in Fig. 1).

Registration module 42 comprises at least one optical marker 50 located on a surface 57 of the outer perimeter of shaft 56. Marker 50 is typically obscured by tube 58 and shown in Fig. 2A purely for the sake of description clarity.

In some embodiments, marker 50 and surface 57 have different respective optical attributes. In an embodiment, the reflectivity of marker 50 may be substantially higher than the reflectivity of surface 57. The reflectivity level may be controlled, for example, by applying black coating to surface 57 and white coating to marker 50, or *vice versa.* In another embodiment, marker 50 may have a different topography than surface 57 (e.g., a wedge-shaped protrusion or intrusion) so as to scatter the reflected light in non-orthogonal direction, thus differs in reflection from surface 57.

In some embodiments, six substantially identical optical devices 60A - 60F are mounted around a surface 59, which is the inner perimeter of insertion tube 58. The inner perimeter, on which the optical devices are positioned, faces marker 50 as depicted in FIGs. 2A and 2B.

In the example of Fig. 2A, only devices 60A, 60B, 60C and 60D are shown since devices 60E and 60F are located in the rear side of tube 58. Tube 58 is typically opaque, thus, optical devices 60A-60F are obscured from the direction of y-axis, and shown in Fig. 2A purely for the sake of clarity. In other embodiments, the optical devices may be located at any suitable location of the distal end that enables the devices to detect optical reflections from optical marker 50.

In yet other embodiments, the number of optical devices may comprise any suitable number (other than six) that provides the angular position of the marker with respect to opening 44. In some embodiments, more than one marker 50 is used.

In an embodiment, device 60A comprises a light source 52A, such as a light emitting diode (LED) or any other suitable light source. Device 60A further comprises a detector 54A located in close proximity to light source 54. The light source is configured to emit light toward surface 57, and detector 54A is configured to detect light reflected from surface 57.

In some embodiments, as shaft 56 rotates, all optical devices (e.g., 60A-60F) illuminate surface 57 and detect the corresponding reflected light. For example, when marker 50 passes in front of device 60A, light source 52A illuminates marker 50 and detector 54A detects the light reflected from the marker and outputs a short duration signal indicative of the detected passing marker, via cable 32, to processor 34. Typically, only one detector indicates positive detection of the marker at a given time. Thus, other detectors (e.g., 54B-54F) also output their corresponding signals from surface 57 to processor 34, however, none of their signals comprises indicative signals of marker 50.

When shaft 56 is stationary with marker 50 facing one of the optical devices, the detector of that optical device constantly detects marker 50 while all other detectors output signals from surface 57 without indication of marker 50.

Referring to FIG. 2B, which is a schematic sectional view of registration module 42 in a y-z plane, in accordance with an embodiment of the present invention. In an embodiment, shaft 56 rotates counterclockwise so that in the example of Fig. 2B processor 34 received the most recent signal indicating the location of marker 50 from device 60B and is expected to receive the subsequent corresponding signal from device 60C. In an embodiment, marker 50 is located along distal end 38, at a predefined angular displacement (e.g., 90°) with respect to cutter 46. Processor 34 takes this predefined angular displacement into account in calculating the angular position of cutter 46 relative to opening 44.

Processor 34 is configured to receive the detected signals from detectors 52A-52F (including the indicative signals of the marker) and to calculate and display the rotational angle of cutter 46 relative to opening 44. In an embodiment, processor 34 may display a numerical value of the rotational angle. In another embodiment, processor 34 may use the calculated rotational angle to determine and display the status of opening 44, e.g., whether opening 44 is open, closed or partially open. Furthermore, processor 34 may display an icon that indicates the status of opening 44 on display 36. Any other suitable visualization or presentation can be used in alternative embodiments.

In some embodiments, physician 24 may use proximal end 30 to rotate shaft 56 so as to control the location of cutter 46 relative to opening 44. For example, after concluding the removal of polyp 45, physician 24 may rotate shaft 56 so as to block opening 44 before retracting catheter 28 out of nose 26. In an alternative embodiment, processor 34 may control proximal end 30 so as to automatically rotate shaft 56, based on the signals received from the optical devices. For example, before cutting polyp 45 processor 34 may calculate the angular position of cutter 46 relative to opening 44. In some cases, e.g., when opening 44 is closed or partially open, processor 34 may rotate shaft 56 so as to obtain a fully open position of opening 44. Similarly, processor 34 may automatically rotate the shaft so as to close opening 44 before extracting catheter 28.

The examples of FIGs. 1, 2A and 2B refer to a specific configuration of registration module 42 (e.g., marker, light source and detector), chosen purely for the sake of conceptual clarity. In alternative embodiments, the disclosed techniques can be used, *mutatis mutandis,* in various other types of surgical, diagnostic and therapeutic catheters. It will be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

## Claims

1. A surgical apparatus, comprising:
a rotatable shaft, which comprises at least one optical marker located thereon;
a hollow insertion tube for insertion into a patient body, the tube coaxially disposed around the shaft; and
one or more optical devices, which are coupled around an inner perimeter of the insertion tube and are configured to detect the marker and output one or more signals indicative of an angular position of the detected marker.

2. The apparatus according to claim 1, and comprising a processor that is configured to receive the signals from the optical devices and to calculate, based on the signals, a rotational angle of the shaft with respect to the insertion tube.

3. The apparatus according to claim 2, wherein the processor is further configured to automatically rotate the shaft, in response to the signals received from the optical devices, so as to set a desired rotational angle of the shaft with respect to the insertion tube.

4. A method, comprising:
providing a surgical tool comprising a rotatable shaft, which comprises at least one optical marker located thereon, and a hollow insertion tube coaxially disposed around the shaft;
inserting the surgical tool into a patient body;
while the surgical tool is inside the patient body, detecting the marker using one or more optical devices that are coupled around an inner perimeter of the insertion tube; and
outputting by the optical devices one or more signals indicative of an angular position of the detected marker.

5. The method according to claim 4, and comprising receiving the signals from the optical devices and calculating, based on the signals, a rotational angle of the shaft with respect to the insertion tube.

6. The method according to claim 5, and comprising automatically rotating the shaft, in response to the signals received from the optical devices, so as to set a desired rotational angle of the shaft with respect to the insertion tube.

7. A method for producing a surgical tool, the method comprising:
providing a rotatable shaft, which comprises at least one optical marker located thereon;
disposing a hollow insertion tube coaxially around the shaft; and
coupling one or more optical devices around an inner perimeter of the insertion tube.

8. The apparatus according to claim 1 or the method according to claim 4 or claim 7, wherein the hollow insertion tube has an opening, and wherein the rotatable shaft comprises a cutter, which is configured, when the shaft is rotating, to cut an object that enters the insertion tube via the opening.

9. The apparatus or the method according to claim 8 wherein the marker is located at a predefined rotational angle with respect to the cutter.

10. The apparatus according to claim 1 or the method according to claim 4 or claim 7, wherein the marker faces the inner perimeter of the insertion tube.

11. The apparatus according to claim 1 or the method according to claim 4 or 7, wherein each of the optical devices comprises a light source and a detector, wherein the light source is configured to illuminate the inner perimeter, and wherein the detector is configured to detect the marker by detecting a light reflection from the inner surface.

12. The apparatus or the method according to claim 11, wherein the light source comprises a light emitting diode (LED).
